(19) Europäisches Patentamt
European Patent Office
Office européen des brevets

(11) Veröffentlichungsnummer : **0 329 961 B1**

(12) # EUROPÄISCHE PATENTSCHRIFT

(45) Veröffentlichungstag der Patentschrift : **24.06.92 Patentblatt 92/26**

(51) Int. Cl.$^5$ : **A61K 49/00,** G01N 33/50

(21) Anmeldenummer : **89101215.5**

(22) Anmeldetag : **25.01.89**

(54) **Verwendung von 2-Oxo-pyrrolidin-1-acetamid zur Bestimmung der glomerulären Filtrationsrate beim Menschen.**

(30) Priorität : **02.02.88 DE 3802996**

(43) Veröffentlichungstag der Anmeldung : **30.08.89 Patentblatt 89/35**

(45) Bekanntmachung des Hinweises auf die Patenterteilung : **24.06.92 Patentblatt 92/26**

(84) Benannte Vertragsstaaten : **AT BE CH DE ES FR GB IT LI NL SE**

(56) Entgegenhaltungen :
**L. THOMAS: "Labor und Diagnose", 3. Auflage, 1988, Seiten 406-442, Die Medizinische Verlagsgesellschaft, Marburg, DE; L.THOMAS et al.: "Niere und Harnwege"**

(73) Patentinhaber : **CASSELLA Aktiengesellschaft
Hanauer Landstrasse 526
W-6000 Frankfurt am Main 61 (DE)**

(72) Erfinder : **Schultz, H.-U., Prof. Dr. med. habil.
Bahnhofstrasse 8
W-2407 Bad Schwartau (DE)**

(74) Vertreter : **Urbach, Hans-Georg, Dr. et al
Hanauer Landstrasse 526
W-6000 Frankfurt am Main 61 (DE)**

EP 0 329 961 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft die Verwendung von 2-Oxo-pyrrolidin-1-acetamid (Piracetam) der Formel I

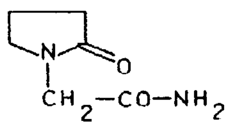

(I)

zur Bestimmung der glomerulären Filtrationsrate beim Menschen, sowie ein Verfahren zur Bestimmung der glomerulären Filtrationsrate beim Menschen.

Im Zusammenhang mit diagnostischen und therapeutischen Maßnahmen im präklinischen und klinischen Bereich kommt der Bestimmung der glomerulären Filtrationsrate eine große Bedeutung zu, weil sich mit ihrer Hilfe die Einschränkung der Nierenfunktion generell abschätzen läßt. Unter der glomerulären Filtrationsrate (GFR) versteht man die von allen Glomerula beider Nieren pro Zeiteinheit produzierte Menge des Primärharns, der das Ultrafiltrat des Blutes darstellt. Diese glomeruläre Filtrationsrate kann grundsätzlich mit jedem Stoff ermittelt werden, welcher vollständig ultrafiltriert wird, d.h. im Primärharn in gleicher Konzentration erscheint wie im Blut und überdies weder tubulär sezerniert noch aus dem Primärharn rückresorbiert wird.

Zum Zwecke der Quantifizierung der glomerulären Filtrationsrate wird die Clearance der jeweils verwendeten Testsubstanz untersucht, wobei gemäß der Definition von van Slyke der Begriff der Clearance als diejenige virtuelle Plasmamenge in ml definiert wird, die in den Nieren in der Zeiteinheit (min) von einer bestimmten Substanz befreit wird.

In der Vergangenheit sind die verschiedensten Clearance-Methoden angewendet worden, von denen beispielsweise die endogene Kreatinin-Clearance, die Inulin-Clearance oder die $^{51}Cr$-EDTA ($Na_2{}^{51}Cr$-ethylendiamintetraacetat)-Clearance Bedeutung erlangten, die aber auch mit einer Reihe von Nachteilen behaftet sind (z.B. Henning, Klinische Laboratoriumsdiagnostik, Urban & Schwarzenberg, München und Berlin 1960).

So ist je nach Ausführungsart der jeweiligen Clearance-Bestimmung eine Dauerinfusion der Clearancesubstanz und/oder ein mit unangenehmer Katheterisierung verbundenes Sammeln von Urin und/oder eine mehrmalige Blutentnahme nötig.

Die Verwendung radioaktiver Testsubstanzen belastet den Organismus zusätzlich.

Ein weiterer Nachteil ist darin zu sehen, daß viele Testsubstanzen von körpereigenem Eiweiß gebunden werden, so daß bei der Berechnung der Clearance ein auf Erfahrung beruhender Korrekturfaktor berücksichtigt werden muß. Dies trifft beispielsweise auch auf die in der DE-A 32 24 641 beschriebene Methode zu, die sich als Testsubstanz eines Corrinoids, insbesondere des Vitamin $B_{12}$, bedient.

2-Oxo-pyrrolidin-1-acetamid (Piracetam) ist bekannt und als nootropes Pharmakon im Handel.

Es wurde nun überraschenderweise gefunden, daß die Verwendung von 2-Oxo-pyrrolidin-1-acetamid zur Bestimmung der glomerulären Filtrationsrate gegenüber dem Stand der Technik erhebliche Vorteile aufweist.

Die Ermittlung der GFR kann allein mit Hilfe von Plasmaspiegelbestimmungen erfolgen, wobei folgendes Verfahren angewendet wird.

1. Applikation einer Testdosis von 2-Oxo-pyrrolidin-1-acetamid.
2. Nach einer Latenzzeit von 2 Stunden Durchführung von zwei zeitdifferenten Blutabnahmen.
3. Konzentrationsmessung von 2-Oxo-pyrrolidin-1-acetamid in den erhaltenen Serumproben und Erstellen einer Konzentrations/Zeit-Kurve in halblogarithmischem Maßstab (Blutspiegelkurve).
4. Berechnung der Clearance.

Die Testdosis kann sowohl injiziert als auch oral verabreicht werden. Üblicherweise werden Mengen von 800 bis 3000 mg gegeben.

Bei der Blutabnahme erfolgt die Zweitabnahme normalerweise 2 bis 3 Stunden nach der Erstabnahme. Es genügen jeweils 1 bis 2 ml Blut. Die Konzentrationsbestimmung der Testsubstanz erfolgt nach Extraktion aus dem Serum, vorteilhafterweise gaschromatographisch nach einer modifizierten Methode von C. Hesse und M. Schulz (Chromatographia Vol. 12, No. 1 (1979) 12-16).

Die Clearance wird vorteilhafterweise nach folgender Formel berechnet:

$$Cl_{tot} = \frac{K_e \cdot V_d}{60} \text{ (ml/min)}$$

wobei

$Cl_{tot}$ = Gesamtkörperclearance

$K_e$ = Eliminationskonstante und

$V_d$ = Verteilungsvolumen (ml) bedeuten.

Die Eliminationskonstante $K_e$ entspricht der Geschwindigkeitskonstanten des Gesamtprozesses der Elimination aus der Blutbahn. Man erhält sie aus den im halblogarithmischen Maßstab eingetragenen Konzentrationszeitwerten nach folgender Formel:

$$K_e = \frac{\ln c_1 - \ln c_2}{t_2 - t_1} \text{ (1/Zeit)}$$

Der in einer systematischen Untersuchung ermittelte $K_e$-Wert für 2-Oxo-pyrrolidin-1-acetamid beträgt bei männlichen, gesunden Probanden 0,1398 1/h.

Die Ermittlung des Verteilungsvolumens $V_d$ der Substanz im menschlichen Organismus wird nach folgender Formel vorgenommen:

$$V_d = \frac{D}{C_o} \text{ (ml)}$$

worin

D = Dosis der verabreichten Testsubstanz und

$C_o$ = Blutspiegel zur Zeit t = O

bedeutet.

Die Größe $C_o$ dem Schnittpunkt der abfallenden Blutspiegelkurve im halblogarithmischen Maßstab mit der y-Achse zum Zeitpunkt t = 0.

Die Verwendung von 2-Oxo-pyrrolidin-1-acetamid liefert Clearance-Werte, die den mit der Kreatinin- oder Inulin-Clearance ermittelten entsprechen. Gleichzeitig bietet sie gegenüber den bisher bekannten Methoden erhebliche Vorteile. So wird das aufwendige, auch unter klinischen Bedingungen mit Unsicherheiten behaftete Urinsammeln umgangen. Es müssen lediglich zwei Blutproben von je 1-2 ml entnommen werden. Die Testsubstanz kann auch oral verabreicht werden.

Von besonderem Vorteil ist, daß keine Interaktion der Testsubstanz mit anderen Substanzen, wie z.B. Arzneimitteln bei medikamentös behandelten Patienten auftritt. Diese Eigenschaft ist insbesondere auch bei der analytischen Bestimmung hilfreich.

Schließlich fällt auch eine radioaktive Belastung des Organismus weg.

Insgesamt zeichnet sich die Erfindung durch eine hohe Praktikabilität aus und stellt somit eine Bereicherung für die Nierendiagnostik dar.

Beispiel

Einem Kollektiv von zehn jugendlichen männlichen Probanden wurde je 1200 mg 2-Oxo-pyrrolidin-1-acetamid injiziert. Nach 2 Stunden wurde je eine Blutprobe von etwa 2 ml entnommen und nach weiteren 2,5 Stunden eine weitere Probe von wiederum etwa 2 ml. Die Konzentration der Testsubstanz in dem Blutproben wurde folgendermaßen bestimmt:

100 µl Serumprobe wurden mit einem internen Standard von 2-Pyrrolidon-Propionamid sowie mit 1 ml Aceton versetzt und 20 min in einem Rotamixer gemischt. Dann wurde 2 min zentrifugiert, der Überstand dekantiert und bei 62°C unter $N_2$ evaporiert. Dann wurden 100 µl Aceton hinzugefügt und 30 sec im Rotamixer gemischt. Von dieser Probe wurden 1,5 µl in den Gaschromatographen injiziert.

Gearbeitet wurde mit dem Gaschromatographen Sichromat 1 der Firma Siemens AG, ausgerüstet mit einem "on column" Injektor, einem $N_2$-selektiven Detektor und einem Integrator Siemens Typ BD 60. Als Trennsäule wurde eine Glassäule I.D. 2 mm, A.D. 6 mm mit einer Länge von 340 mm verwendet, wobei als stationäre Phase Gaschrom Q, 120-140 mesh, beschichtet mit 10% Carbowax 20 M der Firma WGA Werner Günther Analysentechnik, Pfungstadt, zum Einsatz kam.

Es wurde unter folgenden Bedingungen gearbeitet:

| | | |
|---|---|---|
| Temperaturen: | Ofen: | 220°C |
| | Injektor: | 230°C |
| | Detektor: | 300°C |
| | Detektorstrom: | 770 mA |
| Gase: | Helium: | 45 ml/min |
| | Wasserstoff: | 12 ml/min |
| | Brennluft: | 100 ml/min |

Aus dem so erhaltenen Chromatogrammen, die von hoher Qualität sind und keine Interferenzpeaks aufweisen, lassen sich die Konzentrationen an Testsubstanz ermitteln. Das Peak-Flächen-Verhältnis der Testsubstanz zum internen Standard ist über einen weiten Konzentrationsbereich liner. Es wurden Blutspiegelkurven angefertigt und daraus $C_0$ bestimmt.

Schließlich wurde die Clearance nach oben angegebener Formel berechnet. In Tabelle 1 sind die Ergebnisse der gleichzeitig bestimmen Kreatinin-Clearance gegenübergestellt.

| Proband | Kreatinin-Clearance (ml/min) | 2-Oxo-pyrrolidin-1-acetamid-Clearance (ml/min) |
|---|---|---|
| 1 | 125,9 | 120,93 |
| 2 | 137,75 | 123,53 |
| 3 | 156,64 | 151,90 |
| 4 | 107,90 | 116,91 |
| 5 | 127,83 | 127,38 |
| 6 | 131,74 | 130,97 |
| 7 | 135,44 | 118,06 |
| 8 | 128,68 | 145,84 |
| 9 | 121,08 | 136,66 |
| 10 | 156,47 | 131,08 |
| Durchschnitt | 141,30 | 134,95 |
| Standard-abweichung | 23,94 | 15,17 |

Die mit beiden Methoden ermittelten Werte für die glomeruläre Filtrationsrate unterscheiden sich im Rahmen der Versuchsgenauigkeit nicht voneinander und entsprechen den mit der Inulin-Clearance gemessenen Mittelwerten für gesunde männliche Versuchspersonen (Goldring, W., Chassis, H.: Hypertension and hypertensive disease. The Commonwealth Fund, New York, 1944).

## Patentansprüche

1. Verwendung von 2-Oxo-pyrrolidin-1-acetamid (Piracetam) der Formel I

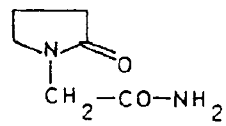

(I)

zur Bestimmung der glomerulären Filtrationsrate beim Menschen.

2. Verfahren zur Bestimmung der glomerulären Filtrationsrate beim Menschen, dadurch gekennzeichnet, daß

1) eine Testdosis von 2-Oxopyrrolidin-1-acetamid appliziert wird,
2) nach einer Latenzzeit von 2 Studen zwei zeitdifferente Blutabnahmen durchgeführt werden,
3) in den erhaltenen Serumproben die Konzentrationen von 2-Oxo-pyrrolidin-1-acetamid bestimmt werden und eine Konzentrations/Zeit-Kurve in halblogarithmischen Maßstab (Blutspiegelkurve) erstellt wird und
4) die Clearance berechnet wird.

## Claims

1. Use of 2-oxo-1-pyrrolidineacetamide (piracetam) of the formula I

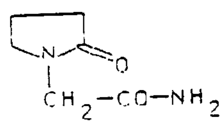

(I)

for the determination of the glomerular filtration rate in humans.

2. Method for the determination of the glomerular filtration rate in humans, characterized in that

1) a test dose of 2-oxo-1-pyrrolidineacetamide is administered,
2) after a latency time of 2 hours, two blood samplings at different times are carried out,
3) the concentrations of 2-oxo-1-pyrrolidineacetamide in the resulting serum samples are measured, and a concentration/time plot on a semilogarithmic scale is constructed (blood level plot) and
4) the clearance is calculated.

## Revendications

1. Utilisation d'oxo-2 pyrrolidine-acétamide-1 (piracétam) de formule I

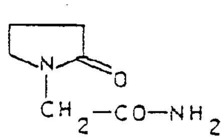

(I)

pour déterminer le taux de filtration glomérulaire chez l'homme.

2. Procédé de détermination du taux de filtration glomérulaire chez l'homme, caractérisé en ce que

1) on applique une dose d'essai d'oxo-2 pyrrolidine-acétamide-1,
2) après un temps de latence de 2 heures, on effectue deux prélèvements sanguins à des instants différents,
3) on détermine les concentrations de l'oxo-2 pyrrolidine-acétamide-1 dans les échantillons de sérum ainsi obtenus, et on établit une courbe concentration/temps sur un papier semi-log (courbe du taux sanguin), et
4) on calcule la clairance.